# EUROPÄISCHE PATENTSCHRIFT

(11) **EP 2 066 310 B1**
(45) Veröffentlichungstag und Bekanntmachung des Hinweises auf die Patenterteilung: **18.04.2012**
(21) Anmeldenummer: 07800646.7
(22) Anmeldetag: 18.09.2007
(51) Int. Cl.: A61K 9/14, A61K 9/51, A61K 9/50, A61K 47/36

(54) **EMULSIONEN ENTHALTEND GUMMI ARABICUM**
EMULSIONS COMPRISING RUBBER ARABICUM
ÉMULSIONS CONTENANT DE LA GOMME ARABIQUE

(30) Priorität: 22.09.2006 CH 15102006
(43) Veröffentlichungstag der Anmeldung: 10.06.2009
(73) Patentinhaber: Mivital AG, 9000 St. Gallen (CH)
(72) Erfinder: STRASSER, Daniel, 9200 Gossau (CH)
(74) Vertreter: Grimm, Siegfried
(86) Internationale Anmeldenummer: PCT/CH2007/000455
(87) Internationale Veröffentlichungsnummer: WO 2008/034273

(56) Entgegenhaltungen:
- EP-A- 0 162 724
- WO-A-97/13503
- WO-A-03/007907
- GB-A- 824 912
- GB-A- 879 325
- JP-A- 59 210 024
- US-A- 4 253 877
- US-A- 4 376 113

## Beschreibung

Die Erfindung betrifft Micellen, micellare Lösungen und Emulsionsvorkonzentrate enthaltend Wirkstoff(e) und Gummi Arabicum; Erzeugnisse enthaltend diese Micellen, micellaren Lösungen bzw. Emulsionsvorkonzentrate, Verfahren zur Herstellung von Micellen, micellaren Lösungen, Emulsionsvorkonzentraten und Erzeugnissen.

Ein typisches Problem bei der Anwendung von Wirkstoffen ist deren unzureichende Bioverfügbarkeit. Unter Bioverfügbarkeit wird der Anteil an Wirkstoff verstanden, der unverändert im systemischen Kreislauf von Mensch / Tier / Pflanze zur Verfügung steht. Sie gibt an, wie schnell und in welchem Umfang der Wirkstoff resorbiert wird und am Wirkort zur Verfügung steht. Bei Wirkstoffen, die intravenös ("i.V.") verabreicht werden, ist die Bioverfügbarkeit definitionsgemäß 100%. Die absolute Bioverfügbarkeit gibt die Bioverfügbarkeit einer auf beliebige Weise applizierten (z. B. peroral) Substanz im Vergleich zur intravenösen Gabe an. Die nach oraler Gabe beobachtete Bioverfügbarkeit nennt man auch orale Bioverfügbarkeit. Insbesondere die Verbesserung der oralen Bioverfügbarkeit ist von Bedeutung, da die orale Verabreichung von Wirkstoffen anderen Darreichungsformen vorzuziehen ist.

Es ist bekannt, dass durch Zugabe von Hilfsstoffen die Bioverfügbarkeit von Wirkstoffen verbessert werden kann. Solche Zusammensetzungen, enthaltend Wirkstoff(e) und Hilfsstoffe(e) werden häufig als "Formulierungen" oder "Wirkstoffformulierungen" bezeichnet. Zahlreiche bekannte Hilfsstoffe, bspw. (Poly)sorbare (Tween ®), wären prinzipiell geeignet, um Formulierungen mit verbesserter Bioverfügbarkeit zu erzeugen. Es hat sich jedoch gezeigt, dass diese Hilfsstoffe wegen anderer Nachteile, z.B. physiologischer Bedenken und/oder unvorteilhaftem Eigengeschmack, wenig geeignet sind.

Weiterhin ist bekannt, dass Gummi Arabicum (E414; CAS.Nr. 9000-01-5, ein bekannter Naturstoff/ Naturstoffgemisch) in Lebensmitteln und bei der Formulierung von Pharmazeutika verwendet wird. Die bekannten Formulierungen können, je nach Art, eine Solubilisierung, Micellierung und / oder Dispergierung bewirken von Wirkstoffen bewirken. In vielen Fällen ist das Ergebnis nicht oder nur teilweise befriedigend. So offenbart GB824912 ein Verfahren zur Stabilisierung von Vitamin E mittels Gummi Arabicum, bei dem intermediär eine Emulsion mit Teilchengrüssen unter 2 um hergestellt wird. Diese Emulsion ist jedoch nicht stabil und wird daher lediglich zur Herstellung eines festen Endproduktes verwendet.

Ferner sind zahlreiche Dokumente bekannt, die spezifische Wirkstoffformulierungen beschreiben. So beschreibt WO97/13503 die Synthese von Wirkstoff-Nanopartikeln mittels Spraytrocknung, US4376113 die Herstellung stabilier Suspensionen und Pulver, GB824912 die Herstellung von Vitamin-E-Zusammensetzungen, WO03/007907 wasserfreie Ubichinon-Konzentrate und deren Herstellung, EP162724 gelartige Zusammensetzungen enthaltend Micellen.

Es ist ferner bekannt, dass Emulsionen - im Gegensatz zu micellaren Lösungen - nur bis zu einer charakteristischen Konzentration der Micellen stabil sind. Unterhalb einer bestimmten Konzentration (critical micelle concentration, cmc) zerfällt die Emulsion und die Phasen scheiden sich. Durch diesen Effekt wird die Bioverfügbarkeit des emulgierten Wirkstoffes auf praktisch 0% verringert.

Bei zahlreichen Wirkstoffen, deren Wirksamkeit bekannt ist, bereitet die Applikation Schwierigkeiten, weil ihre Solubilisierung und Stabilisierung schwierig sind. Genannt sei als Beispiel eines Wirkstoffes das Coenzym Q10. Die Möglichkeit, einen fettlöslichen Wirkstoff dieser oder ähnlicher Qualität in Micellen mit derselben oder auch erhöhten Beladungskapazität zu solubilisieren und gleichzeitig zu stabilisieren würde ungeahnte Perspektiven eröffnen. So könnten derartig behandelte Wirkstoffe oder Medikamente nicht mehr bloß als Kapseln oral verabreicht werden, sondern man könnte die Wirkstoffe einem Getränk beimischen, zum Beispiel einem Sportlergetränk. Das Verfügbarmachen von gezielten, bisher fettlöslichen Wirkstoffen in Getränken eröffnete, dank der Tatsache, dass diese darin löslich wären und außerdem stabil blieben, große Vorteile für den Anwender. Ein weiterer wichtiger Wirkstoff, dessen Applikation sich schwierig gestaltet, ist Insulin. Um eine angemessene Bioverfügbarkeit zu erzielen, muss es i.V. verabreicht werden, was für den Patienten von Nachteil ist. Aus diesem Grund wäre eine orale Darreichungsform wünschenswert. Neben medizinischen Wirkstoffen gibt es eine Reihe weiterer Wirkstoffe, die bisher nicht oder nur ungenügend solubilisiert, dispergiert oder stabilisiert werden konnten.

Neben ungenügender Bioverfügbarkeit sind auch unzureichende Stabilität ("Shelf Life"), ungünstige optische Eigenschaften (wie Trübung und/oder Verfärbung)oder ungünstige physiologische Eigenschaften (wie Nebenwirkungen der Hilfsstoffe, Geruch, Geschmack) der Formulierung bzw. der sie enthaltenden Erzeugnisse zu nennen.

Es besteht daher ein Bedarf, Formulierungen für Wirkstoffe und deren Herstellung zur Verfügung zu stellen, die eines oder mehrere der hier genannten Nachteile lösen. Insbesondere besteht Bedarf an Hilfsstoffen, die Solubilisierungen, Micellierungen und Dispergierungen auslösen, zulassen, unterstützen oder erleichtern. Ideal ist es, wenn solche Hilfsstoffe zugelassen sind (d.h. bereits in Arzneimittel- oder Futtermittel-Listen oder vergleichbaren Registern enthalten sind), weil sie als Unbedenklich klassifiziert sind und keine Einwände oder Vorbehalte seitens Gesundheitsbehörden, Konsumentenorganisationen oder anderen Interessengemeinschaften zu erwarten sind. Doch nicht nur die reine Solubilisierung und Dispergierung eines Wirkstoffes ist von Bedeutung sondern auch die Stabilisierung eines (ggf. solubilisierten und/oder micellierten) Wirkstoffes um eine möglichst lange und vollständig Wirkung bzw. Resorption zu entfalten / verbessern.

Die vorliegende Erfindung betrifft nun ein verbessertes Verfahren, um Wirkstoffe zu solubilisieren, zu dispergieren, zu micellieren und ggf. zu stabilisieren und dadurch Produkte zur Verfügung zu stellen, welche eines oder mehrere der vorstehend genannten Probleme löst. Bei diesen Wirkstoffen handelt es sich zum Beispiel um lipophile Wirkstoffe in hydrophilem Milieu wie auch um hydrophile Stoffe in lipophilem und hydrophilem Milieu, insbesondere im wässrigen Milieu. Mit dem Solubilisieren, Dispergieren und Micellieren von Wirkstoffen wie hier beschrieben wird auch eine Stabilisierung derselben in hydrophilem Milieu erreicht. Schließlich umfasst die Erfindung auch die Verwendung derartiger Stoffe und Wirkstoffe. Die vorliegende Erfindung betrifft weiterhin Wirkstoffe welche in Micellen enthalten sind, Emulsionsvorkonzentrate enthaltend solche Micellen und Erzeugnisse enthaltend Micellen oder Emulsionsvorkonzentrate.

Die vorstehend umrissenen Aufgaben werden gemäss den unabhängigen Ansprüchen gelöst. Die abhängigen Ansprüche stellen vorteilhafte Ausführungsformen dar. Die im Zusammenhang mit der vorliegenden Erfindung gegebenen allgemeinen, bevorzugten und besonders bevorzugten Ausführungsformen, Bereiche können beliebig miteinander kombiniert werden. Ebenso können einzelne Definitionen, Ausführungsformen entfallen bzw. nicht relevant sein. Im folgenden werden verschiedene Aspekte der Erfindung im Detail erläutert.

In einem ersten Aspekt betrifft die Erfindung Micellen mit einem Durchmesser von 2 - 300 nm, enthaltend i) einen oder mehrere Wirkstoffe mit einer Löslichkeit von weniger als 1 g/l, ausgewählt aus der Gruppe der Lebensmittelzusatzstoffe, pharmazeutischen Wirkstoffe, kosmetischen Wirkstoffe, Pflanzenwirkstoffe; ii) Gummi Arabicum; iii) einen oder mehrere Hilfsstoffe aus der Gruppe der Polyole. Es wurde überraschend gefunden, dass Wirkstoffe, welche in solchen Micellen eingebettet sind eine deutlich verbesserte Bioverfügbarkeit aufweisen.

Unter "Wirkstoff" wird im Zusammenhang mit der vorliegenden Erfindung eine natürliche, naturidentische oder synthetische Verbindung verstanden welche eine physiologische Wirkung im/am Menschen, Tier oder Pflanze bewirkt. Wirkstoffe können nach Ihrer Funktion in die Gruppen Lebensmittelzusatzstoffe, pharmazeutischen Wirkstoffe (für Mensch oder Tier), kosmetische Wirkstoffe, Pflanzenwirkstoffe unterteilt werden, wobei einzelne Wirkstoffe ggf. mehreren Gruppen zugeordnet werden können. Im Zusammenhang mit der vorliegenden Erfindung werden bevorzugt solche Wirkstoffe verwendet, welche sich anderweitig schlecht formulieren lassen und/oder eine ungenügende Bioverfügbarkeit haben. Solche Wirkstoffe haben typischerweise eine Wasser-löslichkeit von kleiner als 10 g/l (20°C; neutrales Medium) oder von kleiner als 100 g/l (20°C, physiolog. Medium (z. B. synthic body fluid)). Die Molmasse solcher Wirkstoffe kann in einem breiten Bereich schwanken und umfasst "kleine Moleküle" ab einer Molmasse von etwa 50g/mol bis zu Makromolekülen von ca.50.000g/mol. Der Begriff Wirkstoff soll neben einem einzelnen Wirkstoff auch die Kombination mehrerer Wirkstoffe umfassen, wobei jeder einzelne Wirkstoff einer solchen Kombination die hier dargelegten Eigenschaften aufweist.

"Lebensmittelzusatzstoffe" umfasst solche Verbindungen, die für den menschlichen Verzehr zugelassen sind und Lebensmitteln zugesetzt werden können. Lebensmittelzusatzstoffe sind dem Fachmann bekannt und können z.B. in der Verordnung des EDI über die in Lebensmitten zulässigen Zusatzstoffe (Zusatzstoff-verordnung, ZuV) vom 23. November 2005 identifiziert werden. Im Rahmen der vorliegenden Erfindung sei insbesondere auf die Klasse der Vitamine (z.B. Vitamin A, E), der Fettsäuren (z.B. ungesättigte Fettsäuren, wie Omega-3-Fettsäuren, Omega-6-Fettsäuren), der Enzyme (z.B. aus der Gruppe der Flavinoide) und der Co-Enzyme (z.B. Coenzym Q10) und Farbstoffe (z.B Carotinoide wie beta-Carotin, Lycopen und Lutein) verwiesen.

"Pharmazeutischen Wirkstoffe" umfasst solche Verbindungen, die für die Prävention und Therapie von Krankheiten des Menschen oder Tieres zugelassen sind und/oder zugelassen sind. Pharmazeutische Wirkstoffe sind dem Fachmann bekannt und können z.B. im "Orange Book" oder der "Roten Liste" identifiziert werden. Im Rahmen der vorliegenden Erfindung sei insbesondere auf proteinogene Wirkstoffe (z.B. natürliches oder gentechnisch hergestelltes Insulin) verwiesen.

"Kosmetische Wirkstoffe" umfasst solche Verbindungen, die in der Kosmetik eingesetzt werden und für eine physiologische Wirkung (z.B. auf Haut / Haare / Nägel) oder eine optische Wirkung (z.B. Färbung) verantwortlich sind. Kosmetische Wirkstoffe sind dem Fachmann bekannt und können z.B. nach INCI identifiziert werden. Im Rahmen der vorliegenden Erfindung sei insbesondere auf die Klasse der Cosmeticals verwiesen.

"Pflanzenwirkstoffe" sind solche Verbindungen, die im Bereich der Land- und Forstwirtschaft und des Gartenbaus eingesetzt werden. Pflanzenwirkstoffe sind dem Fachmann bekannt und können z.B. im "Pesticide Manual" identifiziert werden. Im Rahmen der vorliegenden Erfindung sei auf die Gruppe der Herbizide, Fungizide, Insektizide und Wachstumsregulatoren verwiesen.

Es sei darauf verwiesen, dass Wirkstoffe in mehrere der vorstehend genannten Kategorien fallen können; also z.B. Lebensmittelzusatzstoff und Pharmazeutikum (z.B. weil eine Pharmazeutische Wirkung noch nicht erkannt oder strittig ist). Im Rahmen der vorliegenden Erfindung genügt es, wenn der Fachmann einen Wirkstoff, wie vorstehend definiert, zumindest einer dieser Klassen zuordnen kann.

Unter "Gummi Arabicum" wird der bekannte, kommerziell erhältliche Naturstoff, welcher als E414 bzw. CAS 9000-01-05 registriert ist, verstanden. Aufgrund seiner natürlichen Herkunft kann die Zusammensetzung schwanken, wobei die unterschiedlichen Gemische in den verschiedenen Reinheitsgraden umfasst sind. ("natürlicher Gummi Arabicum"). Des weiteren umfasst der Begriff Polyarabinsäure und deren Derivate, insbesondere saure Erdalkali- und Alkalisalze, welche auf synthetischen bzw. halbsynthetischen Weg zugänglich sind. ("synthetischer Gummi Arabicum")

Unter "Hilfsstoffen" werden solche Verbindungen verstanden, die einen positiven Einfluss auf die Micelle, das Emulsionspräkonzentrat und/oder das fertige Erzeugnis haben. Eine Gruppe von Hilfsstoffen sind Lösungsvermittler, welche den Schmelzpunkt von Gummi Arabicum vermindern bzw. mit Gummi Arabicum eine homogene Phase unterhalb dessen Schmelzpunktes bilden. Typische Beispiele sind Verbindungen aus der Gruppe der Polyole, wie z.B. Glyzerin, Propylen-Glykol, Polyethylen-Glykol 400 etc. Bevorzugt werden solche Hilfsstoffe zugesetzt, wenn der zu verarbeitende Wirkstoff am Schmelzpunkt des Gummi Arabicum thermisch labil ist (z.B. Proteine).

Der Begriff "Micelle" ist dem Fachmann bekannt. Er bezeichnet Gebilde mit einem "Kern" und einer "Hülle" in einer bestimmten Grössenordnung und einer Form, die als unregelmässig ellipsoid oder kugelförmig beschrieben wird. Typischerweise haben Micellen einen Durchmesser von unter 500 nm und zeigen aufgrund ihrer Herstellung eine gewisse Grössenverteilung. Grösse und Grössenverteilung kann mit optischen Methoden bestimmt werden (z.B. Mikroskope) Bevorzugt haben Micellen im Rahmen der vorliegenden Erfindung einen Durchmesser von 2-300 nm; besonders bevorzugt 10 - 100 nm. Typischerweise sind mindestens 66%, bevorzugt 75% am meisten bevorzugt 80% der Micellen innerhalb des vorstehend genannten Intervalls. Der Kern der Micelle enthält im Wesentlichen den oder die Wirkstoff(e), die Hülle enthält im wesentlichen Gummi Arabicum. Da eine Micelle ein dynamisches Gebilde ist, sind die Übergänge zwischen Kern/Hülle und Hülle/Umgebung mehr oder weniger scharf abgegrenzt bzw. fliessend. Ggf. vorliegende, weitere Hilfsstoffe befinden sich, in Abhängigkeit von Ihrem Verteilungskoeffizienten, entweder überwiegend im Kern oder überwiegend in der Hülle der Micelle.

In einer weiteren vorteilhaften Ausführungsform betrifft die vorliegende Erfindung Micellen wie vorstehend beschrieben, in welchen der Wirkstoff Coenzym Q 10 und/oder Vitamin E ist.

In einer weiteren vorteilhaften Ausführungsform betrifft die vorliegende Erfindung Micellen wie vorstehend beschrieben, in welchen der Wirkstoff Insulin ist.

In einer weiteren vorteilhaften Ausführungsform betrifft die vorliegende Erfindung Micellen wie vorstehend beschrieben, in welchen der Wirkstoff Ibedenone ist.

In einer weiteren vorteilhaften Ausführungsform betrifft die vorliegende Erfindung Micellen wie vorstehend beschrieben, in welchen der Wirkstoff Tocotrienol ist. Mit Tocotrienol werden im Rahmen der vorliegenden Erfindung sowohl ein natürlich vorkommendes Gemisch der Tocotrienole als auch ein Gemisch angereichert an einer der Formen alpha, beta, delta, gamma als auch reines alpha-, beta- delta- oder gamma-Tocotrienol verstanden.

In einer weiteren vorteilhaften Ausführungsform betrifft die vorliegende Erfindung Micellen wie vorstehend beschrieben, in welchen der Wirkstoff eine oder mehrere Omega-3-Fettsäuren enthält.

In einer weiteren vorteilhaften Ausführungsform betrifft die vorliegende Erfindung Micellen wie vorstehend beschrieben, enthaltend einen oder mehrere Hilfsstoffe ausgewählt aus der Gruppe der Polyole.

In einer weiteren vorteilhaften Ausführungsform betrifft die vorliegende Erfindung Micellen wie vorstehend beschrieben, in welchem das Verhältnis von Wirkstoff(en) zu Gummi Arabicum im Bereich von 20 : 1 bis 1 : 10 Massen-% , bevorzugt 10:1 bis 1:1 liegt. Generell ist es wünschenswert, die Menge an Gummi Arabicum zu reduzieren und die Menge an Wirkstoffen zu maximieren. Andererseits muss mit Blick auf die angestrebte Verwendung eine ausreichende Stabilität und Bioverfügbarkeit gegeben sein. Das optimale Verhältnis von Wirkstoff(en) zu Gummi Arabicum kann anhand von einfachen Reihenversuchen bestimmt werden. Die Eigenschaften des Wirkstoffs, insbesondere seine Löslichkeit, haben einen Einfluss auf das o.g. Verhältnis. Im Vergleich zu bekannten Wirkstoff-Formulierungen kann mit dem nachstehend beschriebenen Verfahren einerseits das Verhältnis von Wirkstoff(en) zu Gummi Arabicum günstig beeinflusst werden und andererseits eine enge Grössenverteilung der Micellen erzielt werden. Die erfindungsgemässen Micellen sind daher stabil, ermöglichen eine hohe Bioverfügbarkeit und eine günstiges Freisetzungsprofil des Wirkstoffs.

In einem zweiten Aspekt betrifft die Erfindung Emulsionsvorkonzentrate bzw. micellare Lösungen enthaltend i) eine lipohile Phase, welche Micellen wie vorstehend beschrieben enthält, und ii) eine wässrige Phase. Typischerweise sind Micellen "an sich" nicht beständig sondern in einer weiteren Phase dispergiert. Häufig fallen solche Emulsionsvorkonzentrate / micellaren Lösungen direkt bei der Herstellung an. Solche Emulsionsvorkonzentrate / micellaren Lösungen können als Handelsprodukte an die weiterverabeitende Industrie abgegeben werden (zur Herstellung von "Erzeugnissen, s.u.) oder direkt weiterverarbeitet werden. Demgemäss ist die Verwendung von Emulsionsvorkonzentraten / micellaren Lösungen zur Herstellung von Erzeugnissen wie nachstehend definiert mit umfasst.

Emulsionsvorkonzentrate können durch den Typ der Emulsion, die Micellenkonzentration, -grösse, - grössenverteilung charakterisiert werden. Typ: Emulsionsvorkonzentrate können als Öl in Wasser (O/W; die homogene Phase ist wässrig) oder Wasser in Öl (W/O; die disperse Phase ist wässrig) charakterisiert werden. Bevorzugt sind O/W-Emulsionen. Konzentration: Typischerweise ist in Emulsions-vorkonzentraten die Micellen-Konzentration höher als in den Erzeugnissen. Für die vorliegende Erfindung hat sich das Verhältnis von lipophiler Phase zu wässriger Phase im Bereich von 1 : 10 bis 1:0.5 als günstig erwiesen. Grösse und Grössenverteilung der Micellen wurden bereits im Zusammenhang mit der Beschreibung der Micelle erläutert, worauf hiermit verwiesen wird.

In einer vorteilhaften Ausführungsform betrifft die vorliegende Erfindung daher ein Emulsionsvorkonzentrat bzw. eine micellare Lösung wie vorstehend beschrieben, in welchem das Verhältnis von lipophiler Phase zu wässriger Phase im Bereich von 1 : 10 bis 1:1 liegt.

Die "lipophile Phase" der Emulsionsvorkonzentrate bzw. micellaren Lösungen besteht im Wesentlichen, bevorzugt ausschliesslich, aus Micellen wie hier beschrieben.

In einer Ausführungsform besteht die "wässrige Phase" (nur) aus Wasser. Aufgrund der Thermodynamik wird die wässrige Phase stets Gummi Arabicum und Wirkstoff und ggf. weitere in der Micelle vorhandene Hilfsstoffe zu einem gewissen Teil enthalten; der Einfachheit wegen wird dennoch auf eine "reine" wässrige Phase verwiesen. Das verwendete Wasser kann verschiedene Reinheitsgrade (z.B. gereinigt, deionisiert, für i.V. Anwendungen, ...) aufweisen, abhängig von der beabsichtigten weiteren Verwendung. Diese Reinheitsgrade sind von der Erfindung umfasst.

In einer weiteren Ausführungsform enthält die wässrige Phase weitere Komponenten. Solche Komponenten können zur Beeinflussung des pH-Wertes (Säuren, Basen, Puffer), zur Beeinflussung der Ionenstärke (Puffer, Salze) oder zur Beeinflussung von rheologischen Eigenschaften (Verdicker) dienen. Es können eine oder mehrere Komponenten zugegen sein. Solche Komponenten sind dem Fachmann bekannt und können z.B. in Fiedler, Lexikon der Hilfsstoffe für Pharmazie, Kosmetik und angrenzende Gebiete (1989) identifiziert werden.

In einer weiteren Ausführungsform betrifft die Erfindung ein Emulsionsvorkonzentrat, bestehend aus einem transparenten Gel auf der Basis von wenigstens einem Gummi arabicum und einem darin solubilisierten und micellierten Wirkstoff, dessen Konsistenz zwischen halbfest ("Aspikartig") bis flüssig liegt.

In einem dritten Aspekt betrifft die Erfindung Erzeugnisse aus der Gruppe der Lebensmittel, Kosmetika, Pharmazeutika und Pflanzenschutzmittel welches Micellen wie vorstehend beschrieben oder ein oder mehrere Emulsionsvorkonzentrate wie vorstehend beschrieben oder ein oder mehrere micellare Lösungen wie vorstehend beschrieben, enthält.

Solche Erzeugnisse können die unterschiedlichsten Formen aufweisen. Zur Illustration seien die folgenden Erzeugnisse aufgeführt. i) Flüssigkeiten: als Getränk im Lebensmittel-Bereich; Lösung, Tropfen, Sirup im pharmazeutischen Bereich; als Spray oder Lösung im kosmetischen Bereich, als Sprühlösung im Agro-Bereich. ii) Gele oder Gelees: als Brotaufstrich, zum Auftragen auf den Körper (Pharma, Kosmetik. iii)Cremes oder Pasten: In Backwaren, als Brotaufstrich, in Snacks; als Creme (Pharma, Kosmetik). Wie aus vorstehender Aufzählung deutlich wird, kann das Erzeugnis das fertige Marktprodukt sein (z.B. bei Flüssigkeiten) oder Teil eines Marktproduktes sein (z.B. die Creme in einer Backware)

Erzeugnissen wie vorstehend beschrieben kann das Emulsionsvorkonzentrat / die micellare Lösung in unterschiedlichen Mengen beigefügt werden. Die Menge ist u.a. abhängig vom gewünschten Ergebnis / der benötigten Menge an zugeführtem Wirkstoff. Diese Menge kann in einfachen Reihenversuchen ermittelt werden. Da die Emulsionsvorkonzentrate / micellaren Lösungen eine verbesserte Bioverfügbarkeit gewährleisten, kann im Allgemeinen mit einer vergleichsweise geringen Menge gearbeitet werden; was als erheblicher Vorteil betrachtet wird. Es hat sich auch gezeigt, dass aufgrund der Eigenschaften zu praktisch allen Erzeugnissen Emulsionsvorkonzentrate / micellare Lösungen zugesetzt werden können, ohne die Eigenschaften der Erzeugnisse negativ zu beeinflussen. So bleiben Flüssigkeiten im Wesentlichen klar und über lange Zeit stabil. Die Erzeugnisse werden ferner in ihren sensorischen Eigenschaften (insbes. Geschmack) nicht neg. beeinflusst. Ferner sind keine physiologisch nachteiligen Eigenschaften bekannt. Diese positiven Eingenschaften werden aus Sicht des Marketings und Endverbrauchers von Lebensmitteln als wesentlicher Vorteil angesehen.

In einer bevorzugten Ausführungsform betrifft die vorliegende Erfindung ein Erzeugnis aus der Gruppe der Lebensmittel, insbesondere ein Getränk, welches Coenzym Q10 und / oder Vitamin E enthält.

In einer bevorzugten Ausführungsform betrifft die vorliegende Erfindung ein Erzeugnis aus der Gruppe der Lebensmittel, insbesondere ein Getränk, oder der Pharmazeutika, insbesondere eine flüssige, oral verabreichbare Formulierung, welches Ibedenone enthält.

In einer bevorzugten Ausführungsform betrifft die vorliegende Erfindung ein Erzeugnis aus der Gruppe der Lebensmittel, insbesondere ein Getränk, oder der Pharmazeutika, insbesondere eine flüssige, oral verabreichbare Formulierung, welches Tocotrienol enthält.

In einer bevorzugten Ausführungsform betrifft die vorliegende Erfindung ein Erzeugnis aus der Gruppe der Pharmazeutika, insbesondere eine oral verabreichbare Zusammensetzung, welche Insulin enthält.

In einer weiteren Ausführungsform betrifft die vorliegende Erfindung ein Erzeugnis, enthaltend Gummi arabicum und einem darin solubilisierten, dispergierten und/ oder stabilisierten micellierten Wirkstoff, wobei die Konsistenz des Erzeugnisses zwischen halbfest und flüssig liegt.

In einer weiteren Ausführungsform betrifft die vorliegende Erfindung ein Erzeugnis, bestehend aus einem transparenten Gel welches i) Gummi arabicum und/oder Gummi arabicum / Glycerin-Ester und/oder eines deren Substitute und/oder Derivate, und ii) ein oder mehrere Lösungsmittel aus der Gruppe Wasser, Glyzerin, Propylen-Glykol, Polyethylen-Glykol 400, Ethanol, Macrogol 400, Isopropanol sowie einen oder mehrere darin solubilisierten, dispergierten und stabilisierten lipophilen oder hydrophilen Wirkstoffe enthält.

Die Konsistenz von Erzeugnissen kann in einem weiten Bereich variieren. So sind feste (schnittfeste, brüchige) halb-feste (z.B. Aspik-artige) und flüssige (dünnflüssig wie Wasser oder sirupöse) Erzeugnisse umfasst. Erzeugnisse müssen nicht homogen sein (Cremes, Schäume) oder Können verkapselt sein (Dragees).

In einem vierten Aspekt betrifft die Erfindung ein Verfahren zur Herstellung eines Emulsionsvorkonzentrates / einer micellaren Lösung umfassend die Schritte i)Herstellen einer homogenen Phase entweder durch Aufschmelzen von Gummi Arabicum bei 40 - 60°C oder durch Auflösen von Gummi Arabicum in einem oder mehreren Hilfsstoffen bei 0 - 60°C; ii) dosierte Zugabe von Wirkstoff, welcher ggf. in einer wässrigen Phase dispergiert ist, zur gerührten homogenen Phase; iii) ggf. weitere Zugabe einer wässrigen Phase zur entstandenen gerührten Reaktionsmischung, wobei bei mindestens einem der Reaktionsschritte eine wässrige Phase zugegeben wird und wobei die Temperatur des Wirkstoffs, welcher ggf. in Wasser dispergiert ist, +/-10 °C der homogenen Phase beträgt.

Es hat sich gezeigt, dass nach dem hier beschriebenen Verfahren sowohl Emulsionsvorkonzentrate, micellaren Lösungen auf einfache und zuverlässige Weise für ansonsten schwer formulierbare Wirkstoffe hergestellt werden können. Es ist insbesondere Überraschend, dass sich nach diesem Verfahren micellare Lösungen bilden. Solche micellaren Lösungen sind selbst bei hohen Verdünnungen der Micellen in der kontinuierlichen Phase stabil und stellen dadurch die Bioverfügbarkeit sicher: Bei der Anwendung können die Micellen beliebig mit Wasser oder wässrigen Flässigkeiten (z.B. Magen- und Darmsaft oder Schweiß und interzelluläre Flüssigkeit) verdünnt werden, ohne dass die Emulgatoren von der Oberfläche der Micellen abgelöst werden und dadurch Wasser und Wirkstoff in direkten Kontakt miteinander kommen, was zu einem Brechen der Emulsion führt. Daher kann der Wirkstoff von den Micellen durch Membranen in oraler oder der dermalen Anwendung (Haut und Schleimhaut) intakt an den Wirkungsort transportiert werden. Dieser positive Effekt wird durch die nachfolgend aufgeführten Versuche eindrücklich belegt. Die Möglichkeit, Wirkstoffe in geringen Dosierungen zur Verfügung zu stellen ist wichtig, da viel Wirkstoffe aus Kostengründen nur in geringen Mengen einem Lebensmittel beigegeben werden sollen und / oder weil gesetzliche Vorgaben nur eine bestimmte (maximale) Dosierung erlauben.

Somit betrifft die Erfindung Verfahren zum Solubilisieren, Dispergieren, Micellieren und Stabilisieren von Wirkstoffen, nach dem Verfahren solubilisierte, dispergierte, micellierte und stabilisierte Emulsionsvorkonzentrate, micellaren Lösungen und Erzeugnisse sowie die Verwendung solcher Emulsionsvorkonzentrate und Erzeugnisse.

Unter dem Begriff "Solubilisieren" wird im Zusammenhang mit der vorliegenden Erfindung das Überführen einer lipophilen in eine hydrophile Substanz verstanden.

Unter dem Begriff "Dispergieren" wird im Zusammenhang mit der vorliegenden Erfindung das Vermengen unter Scherkraft von andersartigen/differenten Stoffen verstanden.

Unter dem Begriff "Micellieren" wird im Zusammenhang mit der vorliegenden Erfindung die Überführung von Wirkstoff in Micellen verstanden.

Unter dem Begriff "Stabilisieren" wird im Zusammenhang mit der vorliegenden Erfindung Verfahren verstanden, welches ein System erzeugt in dem Wirkstoff über einen längeren Zeitraum (insbesondere 1 Tag bis 1 Jahr, bevorzugt 5 - 200 Tage) unverändert vorliegt.

Das vorstehend beschriebene Verfahren ist somit geeignet zum Solubilisieren, Dispergieren und Stabilisieren von Wirkstoff in Micellen, dass sich dadurch auszeichnet, dass Gummi arabicum und Wirkstoff innig dispergiert werden, wobei die Mischung danach mit gleichwarmen Wasser überdeckt wird und der sich dadurch spontan bildende Gel homogenisiert wird.

Die Erfindung betrifft insbesondere ein Verfahren wie vorstehend beschrieben, dadurch gekennzeichnet, dass die wässrige Phase aus Wasser besteht.

Die Erfindung betrifft insbesondere ein Verfahren wie vorstehend beschrieben, dadurch gekennzeichnet, dass die Hilfsstoffe aus der Gruppe der Polyole ausgewählt werden.

In einer weiteren Ausführungsform betrifft die vorliegende Erfindung ein Verfahren zum Solubilisieren, Dispergieren, Micellieren und Stabilisieren von Wirkstoffen, in welchem Gummi arabicum eingesetzt wird und der zu behandelnde Wirkstoff innig in bis dahin stehenden Suspension innig dispergiert wird, wobei dieselbe danach mit Wasser der gleichen Temperatur überdeckt wird und das sich dadurch spontan bildende Gel homogenisiert wird.

In einer weiteren Ausführungsform betrifft die vorliegende Erfindung ein Verfahren zum Solubilisieren, Dispergieren, Micellieren und Stabilisieren von Wirkstoffen wie vorstehend beschrieben, dadurch gekennzeichnet, dass Gummi arabicum aufgeschmolzen wird und der zu behandelnde Wirkstoff innig in dieser Suspension dispergiert wird, wobei nach Einbringung des zu behandelnden Stoffes in das Dispergat dasselbe mit Wasser überzogen und das ganze als Einheit wieder homogenisiert wird.

In einer weiteren Ausführungsform betrifft die vorliegende Erfindung ein Verfahren zum Solubilisieren, Dispergieren, Micellieren und Stabilisieren von Wirkstoffen wie vorstehend beschrieben, dadurch gekennzeichnet, dass Gummi arabicum bei Raumtemperatur in einem Hilfsstoff aufgelöst wird, die Temperatur des Dispergates gehalten wird, der Wirkstoff zusammen dispergiert wird, die Schmelze mit Wasser der gleichen Temperatur überzogen wird, die Schmelze homogenisiert wird, wodurch ein leicht trüber bis transparenter Gel entsteht.

In einer weiteren Ausführungsform betrifft die vorliegende Erfindung ein Verfahren zum Solubilisieren, Dispergieren, Micellieren und Stabilisieren von Wirkstoffen wie vorstehend beschrieben, dadurch gekennzeichnet, dass Gummi arabicum bei Raumtemperatur aufgelöst wird, das Gemisch der Stoffe bei gleicher Temperatur durch Zugaben eines oder mehrerer Hilfsstoffe aus der Gruppe umfassend Wasser, Glyzerin, erniedrigt wird, dann der zu solubilisierende, allenfalls thermolabile Wirkstoff, insbesondere Insulin, in der erhaltenen Schmelze dispergiert wird, die Schmelze mit Wasser der gleichen Temperatur überzogen wird, die Schmelze homogenisiert wird, wodurch ein transparentes Gel als entsteht.

Wie aus den vorstehenden Ausführungen deutlich wird, begünstigt eine korrekte Temperaturführung das Verfahren. Mit dem Ausdruck "gleiche Temperatur" und "konstante Temperatur" ist eine im wesentlichen gleiche Temperatur zu verstehen. Diese kann in Abhängigkeit von weiteren Verfahrensparametern, Geräteausführungen und verwendeten Substanzen variieren. Typischerweise ist ein Temperaturintervall von +/-10°C vorteilhaft, besonders vorteilhaft ist ein Temperaturbereich von +/-5°C. Entsprechend bezeichnet Raumtemperatur 22°C +/-10°C, bevorzugt +/-5°C.

Als weiterer wichtiger Verfahrensschritt erweist sich, dass die Gummi arabicum-Schmelze mit dem darin gelösten Wirkstoff sogleich mit einer genügend dicken Schicht Wasser von etwa gleicher Temperatur überdeckt wird. Durch diese Massnahme bildet sich unter dem Wasser von selbst sogleich ein transparentes Gel. Ohne eine solche Überdeckung mit gleichwarmem Wasser härtet die Schmelze aus und ist in dieser Form nicht oder kaum zu applizieren. Die Schmelze sollte daher noch in flüssigem Zustand mit etwa gleichwarmem Wasser überschüttet bzw. überschichtet werden. Mit kaltem Wasser funktioniert die Gelbildung ebenfalls, aber dann wird vor allem eine Dispergierung des Wirkstoffs entstehen. Nachdem das Wasser auf gleicher Temperatur dazugegeben wurde und eine Überdeckung der Schmelze bildet - das Wasser schwimmt nämlich über der Schmelze auftritt die Gel-Bildung ein und der Gel wächst rasch in dem Masse gegen die Wasseroberfläche nach oben, in dem er Wasser aufnimmt. Diese von aussen beobachtbare Gelbildung wird unterstützt durch das rasche in Verbindung bringen von Wasser und Schmelze, etwa durch gezieltes Umrühren. Das Gel nimmt eine mizelläre Struktur an und hat die Konsistenz einer sehr feinen Lösung. Es können so Gele mit Tröpfchendurchmesser von weniger als 40 nm erhalten werden, an welchen Licht nicht daran gebrochen wird und welche daher klar durchsichtig ist. Dies ist umso erstaunlicher, als zum Beispiel 10% eines fettlöslichen Wirkstoffs und ca. 10-20% Gummi arabicum enthalten sind. Diese kleinsten Fetttröpfchen bleiben thermostabil, sodass selbst beim Kochen des Gels keine Vereinigung der Fetttröpfchen resultiert und auch durch die Zugabe von Wasser die micelläre Struktur nicht verändert. Die Konsistenz ist sirupartig oder dünner. Das Gel wird durch Rühren homogenisiert und durch Zugabe von Wasser oder Wasser-Lösungsmittel-Gemischen auf eine geeignete Viskosität verdünnt. Wenn man jedoch mit grossen Scherkräften homogenisiert, so ist das der Gelbildung abträglich. Dann wird das entstehende Gel nicht transparent, was bedeutet, dass neben der Solubilisierung auch eine Dispergierung stattfindet. Wird aber mit normalen Messern gerührt, etwa mit einer Stefan-Rührmaschine, welche eine senkrecht aus dem Behälterboden aufragende Drehachse mit senkrecht dazu angeordneten scharfen Messern aufweist, welche die Rührmasse immer wieder schneiden, oder mit einer Dissolver-Rührmaschine, so ergibt sich sehr schnell ein optisch reiner, schöner und transparenter Gel. In einem solchen geht die Verkeimung wesentlich langsamer vonstatten geht als in einer Flüssigkeit.

Im folgenden wird das Verfahren zum Solubilisieren und Stabilisieren eines Wirkstoffes noch weiter erläutert: Es zeigt sich, dass es einen Unterschied ausmacht, ob man i) Gummi Arabicum lediglich in eine wässrige Phase gibt und dann einen Wirkstoff dazumischt (in der Hoffnung, dass durch eine Homogenisierung der Mischung derselbe geschützt und stabilisiert werde) oder ii) die Verbindung von Gummi Arabicum und dem Wirkstoff in gezielter Weise vorgenommen wird. Ohne sich an eine Theorie gebunden zu fühlen, wird es als besonders wichtig betrachtet, dass sich Gummi Arabicum und der zu behandelnde Wirkstoff auf molekularer Basis zusammenschliessen. Beim einfachen Mischen gemäß i) bleibt der Effekt der Solubilisierung, der Dispergierung und der Stabilisierung bei bloß einigen Prozenten des zugegebenen Wirkstoffes. Wenn Gummi Arabicum nicht mit Lösungsmitteln verunreinigt ist, wird es bei Raumtemperatur flüssig. Es zeigt sich, dass die Mehrheit der lipophilen und hydrophilen Wirkstoffe mit Gummi Arabicum eine homogene Phase bildet. Ohne sich an eine Theorie zu binden, wird diese Art der Solubilisierung (in welcher man Gummi Arabicum auf molekularer oder quasimolekularer Basis mit den zu solubilisierenden Wirkstoff(en) zusammenbringt) als wichtiges Element zur Verbesserung des Verhältnisses von Gummi Arabicum zu Wirkstoff betrachtet. Im Ergebnis kann viel Wirkstoff mit viel weniger Gummi Arabicum umhüllt werden. Damit werden auch Nebenwirkungen von Hilfsstoffen unterdrückt, denn solche werden häufig in großen bis sehr großen Überschuss zum eigentlichen Wirkstoff zugesetzt.

In einem fünften Aspekt betrifft die Erfindung Verfahren zur Herstellung eines Erzeugnisses wie hier beschrieben, dadurch gekennzeichnet, dass ein Emulsionsvorkonzentrat / micellare Lösung wie hier beschrieben mit weiteren Komponenten des Erzeugnisses gemischt wird. Die Zugabe des Emulsions-vorkonzentrats kann auf verschiedenen Stufen bei der Herstellung des Erzeugnisses erfolgen. Das Einbringen und Verteilen von Emulsionsvorkonzentrat in das Erzeugnis kann nach bekannten Methoden und mit vorhandenen Geräten erfolgen. Diese Flexibilität wird als Vorteil angesehen. Bei der Herstellung von Getränken kann dies z.B. der letzte Herstellungsschritt sein. Im Allgemeinen werden produktionstechnische Überlegungen bei der Wahl eines geeigneten Herstellverfahrens für das Erzeugnis eine übergeordnete Rolle spielen. Obwohl die Micellen und Emulsionsvorkonzentrate dieser gemäss dieser Erfindung temperaturstabil sind, hat es sich als vorteilhaft erwiesen, das Emulsions-vorkonzentrat nicht über längere Zeit hohen Temperaturen auszusetzen.

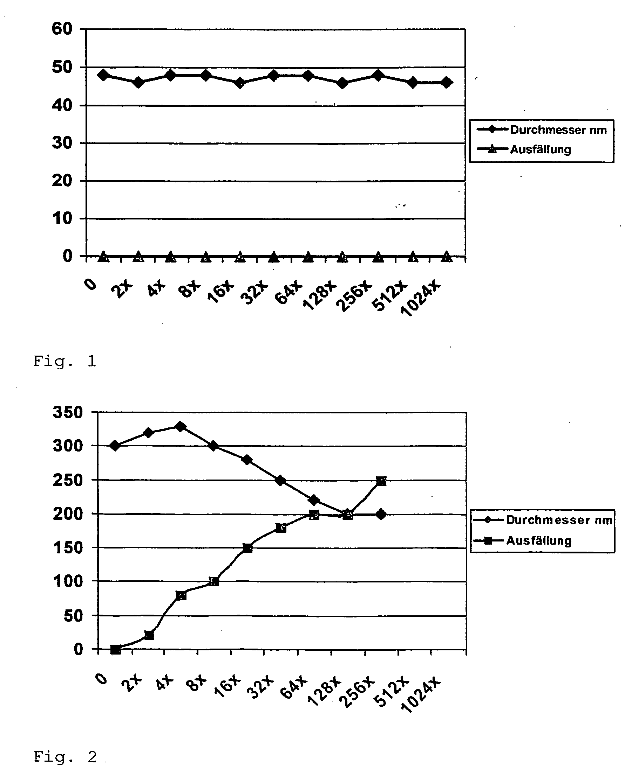

Die im Folgenden dargestellten Beispiele sollen die Erfindung weiter illustrieren, ohne eine Einschränkung darzustellen.
1. Herstellung eines Emulsionsvorkonzentrates: 200 g Gummi Arabicum wird vorgelegt, mit 200 g PEG400 versetzt und bei 40 °C homogenisiert. Eine Suspension aus 20g CoQ10 (Ubichinone) in 200g Wasser wird auf 40°C erwärmt und über einen Zeitraum von 10min unter kräftigem Rühren zugegeben. Anschliessend wird das erhaltene Gemisch mit 380g Wasser von 50°C überschichtet. Die erhaltene Reaktionsmischung ist nach Abkühlung und Entgasung klar und zeigt eine transparente goldgelbe Flüssigkeit.
2. Herstellung eines Erzeugnisses: 1 g Lösung aus der vorherigen Reaktion werden in 500 g kommerzielles Getränk (Limonade der Marke Actilife Q10) gegeben und kurz gerührt. Es ist keine Geschmacksveränderung feststellbar. Das Erzeugnis verändert sich innert 180 Tagen nicht. Die Alterungsprozesse wurden durch beschleunigte Lichtalterung und/oder Temperaturalterung mit anschließendem HPLC bestätigt.
3. Stabilitätsvergleich: Es wurden Coenzym Q10 mit dem vorstehend beschriebenen Solubilisierungsverfahren in micellarer Lösung unter Rühren in einem Becherglas, in einem jeweils mit H2O verdoppelnden Verdünnungsablauf ausgesetzt. Hierbei wurde vor der folgenden Verdünnung bei je einer Probe mittels Mikroskop die Grösse im Durchschnitt 50nm gemessen und auf micellaren Hüllenriss begutachtet. Die Werte in der Figur 1 beziehen sich jeweils auf die micellierte Wirkstoff Konzentration im Umgebungsfluid. Fig. 1 zeigt eine erfindungsgemässe micellare Lösung mit 2% Coenzym Q10 bei neutralem pH; Fig. 2 zeigt im Vergleich dazu eine Emulsion welche identische Komponenten enthält aber nach einem Standardverfahren hergestellt ist, ebenfalls bei neutralem pH. Für die erfindungsgemässe micellare Lösung bleiben die Werte aus Fig. 1 auch nach Absenkung des pH Wertes auf 1.5 identisch, während die Standard-Emulsion bereits bei einem pH von 6.8 zu 100% ausfällt.

Diese Ergebnisse zeigen, dass die nach dem erfindungsgemässen Verfahren hergestellten micellaren Lösungen im Vergleich zu Standard-Emulgierverfahren Produkte liefern, welche gegenüber Verdünnung und pH-Absenkung wesentlich stabiler sind.

## Patentansprüche

1. Zusammensetzung in der Form von Micellen mit einem Durchmesser von 2 - 300 nm, enthaltend
i) einen oder mehrere Wirkstoffe mit einer Wasserlöslichkeit von jeweils weniger als 1 g/l, ausgewählt aus der Gruppe der Lebensmittelzusatzstoffe, pharmazeutischen Wirkstoffe, kosmetische Wirkstoffe, Pflanzenwirkstoffe;
ii) Gummi Arabicum;
i) einen oder mehrere Hilfsstoffe ausgewählt aus der Gruppe der Polyole.

2. Zusammensetzung nach Anspruch 1, enthaltend einen Wirkstoff.

3. Zusammensetzung nach Anspruch 2, in welchem der Wirkstoff Coenzym Q 10 ist.

4. Zusammensetzung nach Anspruch 2 in welchem der Wirkstoff Vitamin E ist.

5. Zusammensetzung nach Anspruch 2 in welchem der Wirkstoff Insulin ist.

6. Zusammensetzung nach Anspruch 1 in welchem der Wirkstoff ausgewählt ist aus der Gruppe umfassend i) Omega-3-Fettsäuren und / oder Omega-6-Fettsäuren ; und / oder ii) Flavinoide; und / oder iii) Carotinoide.

7. Zusammensetzung nach einem der vorhergehenden Ansprüche in welchem das Verhältnis von Wirkstoff(en) zu Gummi Arabicum im Bereich von 20 : 1 bis 1 : 10 Massen-% liegt.

8. Emulsionsvorkonzentrat enthaltend a) eine lipophile Phase, welche eine Zusammensetzung nach einem der vorhergehenden Ansprüche enthält, und b) eine wässrige Phase,
in welchem das Verhältnis von lipophiler Phase zu wässriger Phase im Bereich von 1 : 10 bis 1:0.5 liegt.

9. Micellare Lösung enthaltend a) eine lipophile Phase, welche eine Zusammensetzung nach einem der Ansprüche 1 bis 7 enthält, und b) eine wässrige Phase, in welcher das Verhältnis von lipophiler Phase zu wässriger Phase im Bereich von 1 : 10 bis 1:0.5 liegt.

10. Verfahren zur Herstellung eines Emulsionsvorkonzentrates gemäss Anspruch 8 oder einer micellaren Lösung gemäss Anspruch 9 umfassend die Schritte
i) Herstellen einer homogenen Phase durch entweder durch Aufschmelzen von Gummi Arabicum bei 40 - 60°C oder durch Auflösen von Gummi Arabicum in einem oder mehreren Hilfsstoffen bei 0 - 60°C
ii) dosierte Zugabe von Wirkstoff welcher ggf. in einer wässrigen Phase dispergiert ist, zur gerührten homogenen Phase
iii) ggf. weitere Zugabe einer wässrigen Phase zur entstandenen gerührten Reaktionsmischung,
wobei bei mindestens einem der Reaktionsschritte eine wässrige Phase zugegeben wird und
wobei die Temperatur des Wirkstoffs, welcher ggf. in Wasser dispergiert ist +/-10 °C der homogenen Phase beträgt.

11. Verfahren nach Anspruch 10, **dadurch gekennzeichnet, dass** die wässrige Phase aus Wasser besteht.

12. Verfahren nach Anspruch 10, **dadurch gekennzeichnet, dass** die Hilfsstoffe aus der Gruppe der Polyole ausgewählt werden.

13. Erzeugnis aus der Gruppe der Lebensmittel, Kosmetika, Pharmazeutika und Pflanzenschutzmittel welches eine Zusammensetzung nach Anspruch 1 - 7 oder ein Emulsionsvorkonzentrat nach Anspruch 8 oder eine micellare Lösung nach Anspruch 9 enthält.

14. Erzeugnis gemäss Anspruch 13 aus der Gruppe der Lebensmittel welches Coenzym Q10 enthält oder aus der Gruppe der Pharmazeutika welches Insulin enthält.

15. Verfahren zur Herstellung eines Erzeugnisses nach Anspruch 13 oder 14, **dadurch gekennzeichnet, dass** ein Emulsionsvorkonzentrat gemäss Anspruch 8 mit weiteren Komponenten des Erzeugnisses gemischt wird.

## Claims

1. Composition in the form of micelles having a diameter of 2 - 300 nm, comprising
i. one or more active ingredients each having a water solubility of less than 1 g/l, selected from the group of food additives, pharmaceutically active ingredients, cosmetically active ingredients and agricultural active ingredients;
ii. Gum Arabic;
iii. one or more excipients selected from the group of polyoles.

2. Composition according to claim 1, comprising one active ingredients.

3. Composition according to claim 2, wherein the active ingredient is co-enzyme Q 10.

4. Composition according to claim 2, wherein the active ingredient is vitamin E.

5. Composition according to claim 2, wherein the active ingredient is insulin.

6. Composition according to claim 1, wherein the active ingredient is selected from the group consisting of i) omega-3-fetty acids and / or omega-6-fetty acids; and / or ii) flavonoids; and / or iii) carotenoids.

7. Composition according to any of the preceding claims wherein the ratio of active ingredient(s): Gum Arabic is in the range from 20:1 to 1:10 mass-%.

8. Pre-Concentrate of emulsion comprising a) a lipophilic phase, which comprises a composition according to any of the preceding claims, and b) an aqueous phase, wherein the ratio of lipophilic phase : aqueous phase is in the range of 1 : 10 to 1 : 0.5.

9. Micellar solution comprising a) a lipophilic phase, which comprises a composition according to any of claims 1 to 7, and b) an aqueous phase, wherein the ratio of lipophilic phase : aqueous phase is in the range of 1 : 10 to 1 : 0.5.

10. Process for manufacturing a Pre-Concentrate of emulsion according to claim 8 or a micellar solution according to claim 9 comprising the steps
i. providing a homogeneous phase either by melting Gum Arabic at 40 - 60°C or by dissolving Gum Arabic in one or more excipients at 0 - 60°C
ii. metered addition of active ingredient, which is optionally dispersed in an aqueous phase, to the stirred homogeneous phase
iii. optionally further addition of an aqueous phase to the obtained stirred reaction mixture,
whereby at least in on the reaction steps an aqueous solution is added and whereby the temperature of the active ingredient, which is optionally dispersed in water, is +/-10 °C of the homogeneous phase.

11. Process according to claim 10, **characterized in that** the aqueous phase consists of water.

12. Process according to claim 10, **characterized in that** the excipients are selected from the group of polyoles.

13. Product from the group of foodstuffs, cosmetics, pharmaceuticals and plant protection products which comprises a composition according to claim 1 - 7 or a pre-concentrate of emulsion according to claim 8 or a micellar solution according to claim 9.

14. Product according to claim 13 from the group of foodstuffs which contains co-enzyme Q10 or from the group of pharmaceuticals which contains insulin.

15. Process for manufacturing a product according to claim 13 or 14, **characterized in that** a pre-concentrate of emulsion according to claim 8 is blended with further components of the product.

## Revendications

1. Composition sous la forme de micelles d'un diamètre de 2 à 300 nm, contenant
i) une ou plusieurs substances actives ayant une solubilité dans l'eau de respectivement moins de 1 g/l, choisies dans le groupe des additifs alimentaires, des substances actives pharmaceutiques, des substances actives cosmétiques, des substances actives phytosanitaires ;
ii) de la gomme arabique ;
iii) un ou plusieurs adjuvants choisis dans le groupe des polyols.

2. Composition suivant la revendication 1, contenant une substance active.

3. Composition suivant la revendication 2, dans laquelle la substance active est le coenzyme Q10.

4. Composition suivant la revendication 2, dans laquelle la substance active est la vitamine E.

5. Composition suivant la revendication 2, dans laquelle la substance active est l'insuline.

6. Composition suivant la revendication 1, dans laquelle la substance active est choisie dans le groupe comprenant i) des acides gras oméga-3 et/ou des acides gras oméga-6 ; et/ou ii) des flavinoïdes ; et/ou iii) des carotinoïdes.

7. Composition suivant l'une des revendications précédentes, dans laquelle le rapport de la substance active ou des substances actives à la gomme arabique va de 20:1 à 1:10 en pourcentage en masse.

8. Préconcentré d'émulsion, contenant a) une phase lipophile, qui contient une composition suivant l'une des revendications précédentes, et b) une phase aqueuse, dans lequel le rapport de la phase lipophile à la phase aqueuse va de 1:10 à 1:0,5.

9. Solution micellaire contenant a) une phase lipophile, qui contient une composition suivant l'une des revendications 1 à 7, et b) une phase aqueuse, dans laquelle le rapport de la phase lipophile à la phase aqueuse va de 1:10 à 1:0,5.

10. Procédé de préparation d'un préconcentré d'émulsion suivant la revendication 8 ou d'une solution micellaire suivant la revendication 9, comprenant les stades dans lesquels
i) on prépare une phase homogène par fusion de gomme arabique à 40 à 60°C ou par dissolution de gomme arabique dans un adjuvant ou dans plusieurs adjuvants à 0 à 60°C
ii) on additionne de manière dosée de la substance active, qui, le cas échéant, est dispersée dans une phase aqueuse, en une phase homogène agitée
iii) le cas échéant, on ajoute supplémentairement une phase aqueuse au mélange de réaction agité créé
dans lequel, dans au moins l'un des stades de réaction, on ajoute une phase aqueuse et
dans lequel la température de la substance active, qui est dispersée, le cas échéant, dans de l'eau, est de +/-10°C à celle de la phase homogène.

11. Procédé suivant la revendication 10, **caractérisé en ce que** la phase aqueuse est de l'eau.

12. Procédé suivant la revendication 10, **caractérisé en ce qu'**on choisit les adjuvants dans le groupe des polyols.

13. Produit du groupe des produits alimentaires, des produits cosmétiques, des produits pharmaceutiques ou des agents phytosanitaires, qui contient une composition suivant la revendication 1 à 7 ou un préconcentré d'émulsion suivant la revendication 8 ou une solution micellaire suivant la revendication 9.

14. Produit suivant la revendication 13, choisi dans le groupe des produits alimentaires, qui contient le coenzyme Q10, ou dans le groupe des produits pharmaceutiques, qui contient de l'insuline.

15. Procédé de préparation d'un produit suivant la revendication 13 ou 14, **caractérisé en ce qu'**on mélange un préconcentré d'émulsion suivant la revendication 8 à d'autres constituants du produit.
